(19) Europäisches Patentamt
European Patent Office
Office européen des brevets

(11) Veröffentlichungsnummer : **0 230 401 B1**

(12) # EUROPÄISCHE PATENTSCHRIFT

(45) Veröffentlichungstag der Patentschrift : **18.03.92 Patentblatt 92/12**

(51) Int. Cl.$^5$ : **C07D 207/416,** C08K 5/34

(21) Anmeldenummer : **87810032.0**

(22) Anmeldetag : **19.01.87**

(54) **Aminoxypyrrolidin-2,5-dione.**

(30) Priorität : **24.01.86 US 822179**

(43) Veröffentlichungstag der Anmeldung : **29.07.87 Patentblatt 87/31**

(45) Bekanntmachung des Hinweises auf die Patenterteilung : **18.03.92 Patentblatt 92/12**

(84) Benannte Vertragsstaaten : **DE FR GB IT**

(56) Entgegenhaltungen :
**EP-A- 0 113 318**
**EP-A- 0 138 767**

(73) Patentinhaber : **CIBA-GEIGY AG**
**Klybeckstrasse 141**
**CH-4002 Basel (CH)**

(72) Erfinder : **Pastor, Stephen D.**
**1080 Warburton Avenue Apt. 1C**
**Yonkers New York 10701 (US)**
Erfinder : **Hessell, Edward T.**
**27 North Taylor Avenue**
**Norwalk Connecticut 06854 (US)**

EP 0 230 401 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Aminoxypyrrolidin-2,5-dione, deren Verwendung zum Stabilisieren von organischem Material und das mit deren Hilfe gegen oxidativen, thermischen und/oder aktinischen Abbau stabilisierte organische Material.

Organische Materialien, wie z.B. Kunststoffe und Harze unterliegen thermischem, oxidativem und lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise die Wirksamkeit eines Stabilisators, der die Flüchtigkeit einer Verbindung reduziert, darauf zurückgeführt werden, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenabbruch verhindert. Um die Vergilbung zu unterbinden, müssen Reaktionen, die zu neuen Chromophoren führen, verhindert werden. Weiterhin müssen Probleme der Verarbeitungsstabilität und der Substratverträglichkeit beachtet werden.

Verschiedene organische Hydroxylamine sind bekannt und einige sind im Handel erhältlich. Eine Anzahl von Patenten offenbaren substituierte Hydroxylamine als Antioxidantien für verschiedene Substrate, einschliesslich Polyolefine, Polyester und Polyurethane. Als repräsentative Patente seien die US-Patente 3 432 578, 3 644 278, 3 778 464, 3 408 422, 3 926 909, 4 316 996 und 4 386 224 genannt, welche im wesentlichen N,N-Dialkyl-, N,N-Diaryl- und N,N-Diaralkylhydroxylamine und deren Wirksamkeit als Farbverbesserer beschreiben.

US 4 456 716 offenbart (Hydroxyphenylthio)imide und deren Verwendung als Stabilisatoren für organisches Material. Arylbernsteinsäureimide und deren pharmakologische Aktivität werden in den folgenden "Chemical Abstracts-Zitaten" beschrieben: 93: 8013x (1980), 80: 22481m (1974) und 76: 107806p (1972).

Es wurde nun gefunden, dass die Aminoxypyrrolidin-2,5-dione dieser Erfindung eine Vielzahl der gewünschten Eigenschaften besitzen, wodurch diese Verbindungen zu besonders effektiven Stabilisatoren werden. So schützen sie Polyolefine, Elastomere und Schmiermittel gegen oxidativen und thermischen Abbau. Ferner sind sie sehr wirkungsvolle Farbverbesserer und Verarbeitungsstabilisatoren für Polyolefinzusammensetzungen, die Metallsalze von Fettsäuren und phenolische Antioxidantien enthalten können. Hauptsächlich können die erfindungsgemässen Aminoxypyrrolidin-2,5-dione dazu dienen, Verfärbungen zu vermindern, die auf die Gegenwart von phenolischen Antioxidantien und/oder auf die Verarbeitungsbedingungen zurückzuführen sind. Ferner schützen die erfindungsgemässen Verbindungen das organische Polymer direkt vor den Auswirkungen der Verarbeitungsbedingungen. Sie schützen auch solche Polyolefinzusammensetzungen, die gehinderte Amin-Lichtstabilisatoren oder Kombinationen von phenolischen Antioxidantien und organischen Phosphiten enthalten, vor der Verfärbung. Ferner werden Verfärbungen, die auf die Einwirkung von Zerfallsprodukten natürlicher Gase zurückzuführen sind, merklich vermindert.

Die vorliegende Erfindung betrifft Verbindungen der Formel I,

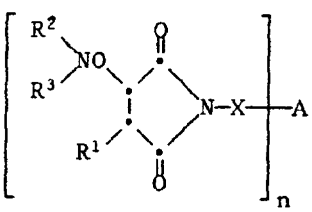

(I)

worin $R^1$ Wasserstoff oder $C_1$-$C_3$-Alkyl bedeutet, $R^2$ und $R^3$ unabhängig voneinander Wasserstoff, $C_1$-$C_{36}$-Alkyl, $C_5$-$C_{12}$-Cycloalkyl, $C_7$-$C_9$-Aralkyl oder durch $C_1$-$C_{36}$-Alkyl substituiertes $C_7$-$C_9$-Aralkyl sind, X eine direkte Bindung oder Phenylen ist, n eine ganze Zahl von 1 bis 3 bedeutet und wenn n 1 ist, A Wasserstoff, $C_1$-$C_{30}$-Alkyl, Aryl, $C_5$-$C_8$-Cycloalkyl, $C_7$-$C_9$-Aralkyl, durch $C_1$-$C_{30}$-Alkyl substituiertes $C_7$-$C_9$-Aralkyl oder 2,5-Dioxo-1H-pyrrol-1-yl ist, wenn n 2 bedeutet, A $C_1$-$C_{12}$-Alkylen, Phenylen, $C_6$-$C_{10}$-Cycloalkylen oder Alkylen-Arylen-Alkylen mit 8 bis 10 Kohlenstoffatomen ist und wenn n 3 bedeutet, A Alkantriyl mit 3 bis 6 Kohlenstoffatomen oder

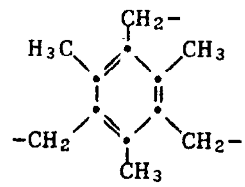

ist.

$R^1$ ist als $C_1$-$C_3$-Alkyl beispielsweise Methyl, Ethyl oder Propyl.

$R^1$ bedeutet bevorzugt Wasserstoff oder Methyl.

Als Beispiele für $R_2$ und $R_3$ als $C_1$-$C_{36}$-Alkyl seien genannt: Methyl, Ethyl, Butyl, Pentyl, 2-Ethylhexyl, Octyl, Decyl, Dodecyl und Octadecyl. $C_1$-$C_{18}$-Alkyl, welches geradkettig oder verzweigt sein kann, ist bevorzugt.

$R^2$ und $R^3$ sind als $C_5$-$C_{12}$-Cycloalkyl beispielsweise Cyclopentyl, Cyclohexyl oder Cyclooctyl. Bevorzugt ist ein Cycloalkylrest mit 5 bis 8 Kohlenstoffatomen. Cyclopentyl und Cyclohexyl sind besonders bevorzugt.

$R^2$ und $R^3$ können als unsubstituiertes oder durch $C_1$-$C_{36}$-Alkyl substituiertes $C_7$-$C_9$-Aralkyl insbesondere Phenylalkyl mit 7 bis 9 Kohlenstoffatomen bedeuten, wobei der Phenylring gegebenenfalls durch $C_1$-$C_{36}$-Alkyl substituiert sein kann. Beispiele sind Benzyl, $\alpha$-Methylbenzyl und $\alpha,\alpha$-Dimethylbenzyl. $R^2$ und $R^3$ sind bevorzugt Benzyl.

Wenn n 1 ist, hat A insbesondere jene Bedeutungen, die oben als Bevorzugungen für $R^2$ und $R^3$ angegeben sind.

A ist als Aryl beispielsweise Naphthyl oder Phenyl, welches gegebenenfalls durch ein bis drei Methylgruppen substituiert sein kann. Typische Beispiele sind: Tolyl, Mesityl, Xylyl und 1- und 2-Naphthyl. Phenyl ist bevorzugt.

A bedeutet als
2,5-Dioxo-1H-pyrrol-1-yl eine Gruppe der Formel

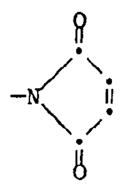 .

A bedeutet als $C_1$-$C_{12}$-Alkylen beispielsweise Methylen, Ethylen oder Hexylen. $C_1$-$C_6$-Alkylen ist bevorzugt.

A ist als Cycloalkylen mit 6 bis 10 Kohlenstoffatomen zum Beispiel Cyclohexylen.

A bedeutet als Alkylen-Arylen-Alkylen mit 8 bis 10 Kohlenstoffatomen insbesondere eine Gruppe der Formel

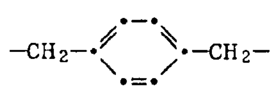 .

A ist bevorzugt Phenylen.

Beispiele für A als $C_3$-$C_6$-Alkantriyl sind die Reste

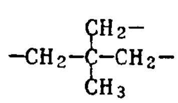 und 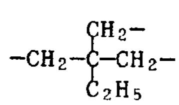 .

Bevorzugt sind Verbindungen der Formel I, worin $R^1$ Wasserstoff oder Methyl ist und $R^2$ und $R^3$ unabhängig voneinander $C_1$-$C_{18}$-Alkyl, $C_5$-$C_6$-Cycloalkyl, Benzyl, $\alpha$-Methylbenzyl oder $\alpha,\alpha$-Dimethylbenzyl bedeuten. n ist bevorzugt 1 oder 2.

Besonders bevorzugt sind Verbindungen der Formel I, worin A $C_1$-$C_{18}$-Alkyl, $C_5$-$C_6$-Cycloalkyl, Benzyl, $\alpha$-Methylbenzyl, $\alpha,\alpha$-Dimethylbenzyl oder Phenyl bedeutet.

A ist bevorzugt $C_1$-$C_6$-Alkylen, Phenylen, Cyclohexylen oder

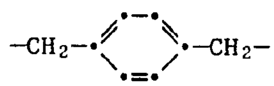 .

Beispiele für bevorzugte Verbindungen der Formel I sind:
N-Phenyl-3-[(N′,N′-dibenzylamino)oxy]pyrrolidin-2,5-dion,
N-n-Octadecyl-3-[(N′,N′-dibenzylamino)oxy]pyrrolidin-2,5-dion,
1,2-Bis[3-[(N,N-dibenzylamino)oxy]-2,5-dioxopyrrolidin-1-yl]benzol,
1-[3-[(N,N-Dibenzylamino)oxy]-2,5-dioxopyrrolidin-1-yl]-2-[2,5-dioxo-1H-pyrrol-1-yl]benzol,
N-Cyclohexyl-3-[(N′,N′-dibenzylamino)oxy]pyrrolidin-2,5-dion,
1,1-Bis[4-[3-[(N,N-dibenzylamino)oxy]pyrrolidin-2,5-dion-1-yl]phenyl]methan,
N-Phenyl-3-[(N′,N′-diethylamino)oxy]pyrrolidin-2,5-dion,
N-Methyl-3-[(N′,N′-dibenzylamino)oxy]pyrrolidin-2,5-dion,
3-[(N,N-dibenzylamino)oxy]pyrrolidin-2,5-dion und
1,6-Bis[3-[(N,N-dibenzylamino)oxy]-2,5-dioxopyrrolidin-1-yl]hexan.

Die Verbindungen der Formel I können in Analogie zu bekannten Verfahren hergestellt werden, beispielsweise durch Umsetzung eines Hydroxylamins der Formel II,

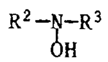 (II)

worin $R^2$ und $R^3$ die oben angegebenen Bedeutungen besitzen, mit einem Maleimid der Formel III,

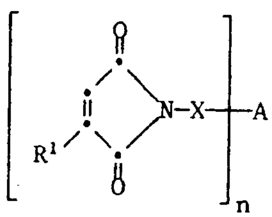 (III)

worin $R^1$, X, A und n die oben angegebenem Bedeutungen haben, in einem geeigneten Lösungsmittel.

Als Lösungsmittel seien genannt: Aromatische Kohlenwasserstoffe, wie beispielsweise Benzol, Toluol, Xylol und dergleichen, oder heterocyclische Ether, wie beispielsweise Tetrahydrofuran. Gegebenenfalls kann als zusätzliches Lösungsmittel ein aliphatischer Alkohol, wie beispielsweise tert-Butanol und dergleichen, eingesetzt werden, so dass ein Lösungsmittelgemisch vorliegt. Die Reaktionstemperatur liegt zweckmässigerweise bei 50° bis 110°C, welches die bevorzugte Rückflusstemperatur des Systems ist.

Die Ausgangsverbindungen der Formeln II und III sind bekannt, teilweise im Handel erhältlich und können in Analogie zu bekannten Verfahren hergestellt werden. Beispielsweise wird in einem Artikel von Hargreaves et al. in "Chemical Reviews 70, 439-469 (1970)" die Herstellung von cyclischen Imiden beschrieben.

Die erfindungsgemässen Verbindungen eignen sich als Stabilisatoren für eine Reihe von organischen Materialien, insbesondere Kunststoffen, Polymeren und Harzen, wie beispielsweise:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere

von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylmitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigem Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den

entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Bevorzugt werden die Verbindungen der Formel I in synthetischen Polymeren, insbesondere Polyolefin-Homopolymeren oder -Copolymeren, eingesetzt.

Besonders bevorzugt werden die erfindungsgemässen Verbindungen in Polyolefinen, wie beispielsweise Polyethylen und Polypropylen, Polystyrol, einschliesslich schlagfestem Polystyrol, Acrylnitril-Butadien-Styrol-Harz (ABS), Styrol-Butadien-Kautschuk (SBR), Isopren als auch natürlichem Kautschuk, Polyester, einschliesslich Polyethylenterephthalat und Polybutadienterephthalat sowie deren Copolymeren, und in Schmierölen, die beispielsweise aus Mineralölen hergestellt werden, eingesetzt.

Im allgemeinen können die erfindungsgemässen Verbindungen in Konzentrationen von ~0,01 bis ~5 Gew.-%, bezogen auf die stabilisierte Zusammensetzung, eingesetzt werden; allerdings kann je nach Verwendung und Substrat die Stabilisatormenge variieren. Bevorzugt verwendet man ~0,5 bis 2 Gew.-%, insbesondere 0,1 bis 1 Gew.-%.

Die Einarbeitung der erfindungsgemässen Stabilisatoren in das organische Material kann nach bekannten Methoden während jeder Verarbeitunbsstufe vor der Formgebung erfolgen. Der Stabilisator kann beispielsweise dem organischen Material in Pulverform zugemischt werden oder eine Emulsion bzw. Suspension des Stabilisators kann mit einer Lösung, Suspension oder Emulsion des organischen Materials vermischt werden.

Die stabilisierten Zusammensetzungen dieser Erfindung können gegebenenfalls auch herkömmliche Additive enthalten.

Beispiele für herkömmliche Additive sind:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-di-methylphenol, 2,6-Di-octadecyl-4-methylphenol, 2,4,6-Tricyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Di-phenyl-4-octadecyloxyphenol.

1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat], Bis-(3-tert.butyl-4-hydroxy-5-methylphenyl)-dicyclopentadien, Bis-[2-(3'-tert.butyl-2'-hydroxy-5'-methyl-benzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)-dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethylisocyanurat, N,N'-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der M-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

## 2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.-amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-ditert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenylundecylketonoxim, Nickelkomplexe des 1-Phenyl-4-lauroyl-5-hydroxypyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, -Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure,

Kondensationsprodukt aus N,N'-Bis-(2,2,6,6-Tetramethyl-4-piperidyl)hexamethylendiamin und 4-tert.Octylamino-2,6-dichlor-1,3,5-s-triazin, Tris-(2,2,6,6-tetramethyl-4-piperidyl)-nitrilotriacetat, Tetrakis(2,2,6,6-tetramethyl-4-piperidyl)-1,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Dioctyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-disubstituierten Oxaniliden.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Verbindungen der Formel I eignen sich sowohl allein als auch in Kombination mit anderen Additiven als Stabilisatoren für eine Reihe von Substraten, insbesondere Polyolefinen, die gegebenenfalls Alkalimetall-, Erdalkalimetall- und Aluminiumsalze von höheren Fettsäuren (Beispiele sind die oben unter Punkt 7 aufgeführten Additive) und/oder gehinderte phenolische Antioxidantien enthalten können. Die erfindungsgemässen Verbindungen vermindern sehr wirksam Verfärbungen, die auf die Anwesenheit von Phenolen zurückzuführen sind. Solche phenolischen Antioxidantien sind beispielsweise: n-Octadecyl-3,5-di-tert-butyl-4-hydroxyhydrocinnamat, Neopentantetrayl- tetrakis(3,5-di-tert-butyl-4-hydroxyhydrocinnamat), Di-n-octadecyl-3,5-di-tert-butyl-4-hydroxybenzyl-phosphonat, 1,3,5-Tris(3,5-di-tert-butyl-4-hydroxybenzyl)isocyanurat, Thiodietbylen-bis (3,5-di-tert-butyl-4-hydroxyhydrocinnamat), 1,3,5-Trimethyl-2,4,6-tris(3,5- di-tertbutyl-4-hydroxybenzyl)benzol, 3,6-Dioxaoctamethylen-bis(3-methyl5-tert-butyl-4-hydroxyhydrocinnamat), 2,6-Di-tert-butyl-p-cresol, 2,2'-Ethyliden-bis(4,6-di-tert-butylphenol), 1,3,5-Tris(2,6-dimethyl-4-tert-butyl-3-hydroxybenzyl)isocyanurat, 1,1,3-Tris(2-methyl4-hydroxy-5-tert-butylphenyl)butan, 1,3,5-Tris[2-(3,5-di-tert-butyl-4-hydroxyhydrocinnamoyloxy) ethyl]isocyanurat, 3,5-Bis(3,5-di-tertbutyl-4-hydroxybenzyl)mesitol, Hexamethylen-bis(3,5-di-tert-butyl-4-hydroxyhydrocinnamat), 1-(3,5-Di-tert-butyl-4-hydroxyanilino)3,5-bis(octylthio)-s-triazine, N,N'-Hexamethylen-bis(3,5-di-tert-butyl-4-hydroxyhydrocinnamamid), Kalzium-bis(ethyl-3,5-di-tert-butyl-4-hydroxybenzyl- phosphonat), Ethylen-bis[3,3-bis(3-tert-butyl-4-hydroxyphenyl)butyrat], Octyl- 3,5-di-tert-butyl-4-hydroxybenzylmercaptoacetat, Bis(3,5- di-tert-butyl-4-hydroxyhydrocinnamoyl)hydrazid und N,N'- Bis[2-(3,5-di-tert-butyl-4-hydroxyhydrocinnamoyloxy)ethyl]oxamid, insbesondere Neopentantetrayl-tetrakis(3,5-di-tert-butyl-4-hydroxyhydrocinnamat), n-Octadecyl-3,5-di-tert-butyl-4-hydroxyhydrocinnamat), 1,3,5-Trimethyl-2,4,6-tris(3,5-di-tert-butyl-4-hydroxybenzyl)benzol, 1,3,5-Tris(3,5-di-tert-butyl-4-hydroxybenzyl)isocyanurat, 2,6-Di-tert-butyl-p-cresol oder 2,2'-Ethylidenbis(4,6-di-tert-butylphenol).

Die Verbindungen der Formel I verhindern auch Verfärbungen, die auf die Anwesenheit von gehinderten Amin- Lichtstabilisatoren zurückzuführen sind, wie beispielsweise Bis(1,2,2,6,6-pentamethyl-4-piperidyl)-2-n-butyl-2-(3,5-di-tert-butyl-4-hydroxybenzyl)malonat, Bis(2,2,6,6-tetramethyl-4-piperidyl)sebacat, Umsetzungsprodukt von Dimethylsuccinat mit 1-Hydroxyethyl-2,2,6,6-tetramethyl-4-hydroxypiperidin, und Umsetzungsprodukt von 2,4-Dichloro-6-octylamino-s-triazin mit N,N'-(2,2,6,6-Tetramethyl-4-piperidyl)hexamethylendiamin.

Die folgenden Beispiele erläutern die Erfindung weiter. Alle Mengenangaben beziehen sich auf das Gewicht, soweit nicht anders angegeben.

Beispiel 1:

Herstellung von N-Phenyl-3-[(N',N'-dibenzylamino)oxy]pyrrolidin-2,5-dion

Eine Suspension von 6,0 g (28 mmol) N,N-Dibenzylhydroxylamin und 4,87 g (28 mmol) N-Phenylmaleimid in 50 ml trockenem Tetrahydrofuran werden 40 Stunden unter Rückfluss erhitzt. Das Lösungsmittel wird im Vakuum entfernt und der Rückstand wird aus Toluol umkristallisiert. Man erhält 6,1 g (56 % der Theorie) eines weissen Feststoffs, der einen Schmelzpunkt von 91-93°C besitzt.

Elementaranalyse:

| | |
|---|---|
| Berechnet für $C_{24}H_{22}N_2O_3$: | C 74,6; H 5,7; N 7,3 |
| Gefunden : | C 74,6; H 5,6; N 7,2 |

Beispiel 2:

Herstellung von N-n-Octadecyl-3-[(N',N'-dibenzylamino)oxy]pyrrolidin-2,5-dion

Die Herstellung erfolgt in Analogie zu Beispiel 1. Es werden 6.1 g (29 mmol) N,N-Dibenzylhydroxylamin und 10,0 g (29,0 mmol) N-n-Octadecylmaleimid eingesetzt.

Der Rückstand wird chromatographisch gereinigt (HPLC; Kieselsäuregel; Eluierungsmittel: Gemisch aus Heptan und Ethylacetat im Verhältnis 9:1). Man erhält 8 g (49 % der Theorie) eines wachsartigen Feststoffs.

Elementaranalyse:

| | |
|---|---|
| Berechnet für $C_{36}H_{54}N_2O_3$: | C 76,8; H 9,7; N 5,0 |
| Gefunden : | C 76,8; H 9,6; N 4,8 |

Beispiel 3:

Herstellung von 1,2-Bis[3-[(N,N-dibenzylamino)oxy]-2,5-dioxopyrrolidin-l-yl)benzol

Die Herstellung erfolgt in Analogie zu Beispiel 1. Es werden 5,33 g (25 mmol) N,N-Dibenzylhydroxylamin und 3,35 g (12,5 mmol) N,N'-ortho-Phenylendimaleimid eingesetzt. Der Rückstand wird chromatographisch gereinigt (HPLC; Kieselsäuregel; Eluierungsmittel: Gemisch aus Heptan und Ethylacetat im Verhältnis 7:3). Man erhält 5,9 g (34 % der Theorie) eines weissen Feststoffs.

Elementaranalyse:

| | |
|---|---|
| Berechnet für $C_{42}H_{38}N_4O_6$: | C 72,6; H 5,5; N 8,1 |
| Gefunden : | C 72,6; H 5,4; N 7,9 |

Beispiel 4:

Herstellung von 1-[3-[(N,N-Dibenzylamino)oxy]-2,5-dioxopyrrolidin-1-yl]-2-(2,5-dioxo-1H-pyrrol-1-yl]benzol

Von dem Rückstand aus Beispiel 3 werden chromatographisch (HPLC) 0,6 g des Monoaddukts isoliert, das als weisser Feststoff vorliegt.

Elementaranalyse:

| | |
|---|---|
| Berechnet für $C_{28}H_{23}N_3O_5$: | C 69,8; H 4,8; N 8,3 |
| Gefunden : | C 70,0; H 5,1; N 8,5 |

Beispiel 5:

Herstellung von N-Cyclohexyl-3-[(N',N'-dibenzylamino)oxy]pyrrolidin-2,5-dion

Die Herstellung erfolgt in Analogie zu Beispiel 1. Es werden 5,33 g (25 mmol) N,N-Dibenzylhydroxylamin und 4,48 g (25 mmol) N-Cyclohexylmaleimid eingesetzt. Der Rückstand wird aus Ethanol umkristallisiert und man erhält 6,0 g (61 % der Theorie) eines weissen Feststoffs mit einem Schmelzpunkt von 75-77°C.

Elementaranalyse:

| | |
|---|---|
| Berechnet für $C_{24}H_{28}N_2O_3$: | C 73,4; H 7,2; N 7,1 |
| Gefunden : | C 73,8; H 7,1; N 7,1 |

Beispiel 6:

Herstellung von Bis[4-[3-((N,N-dibenzylamino)oxy)pyrrolidin-2,5-dion-1-yl]phenyl]methan

Die Herstellung erfolgt in Analogie zu Beispiel 1. Es werden 5,33 g (25 mmol) N,N-Dibenzylhydroxylamin und 4,48 g (12,5 mmol) Bis[4-maleimidophenyl]methan eingesetzt.

Der Rückstand wird chromatographisch gereinigt (HPLC; Kieselsäuregel; Eluierungsmittel: Gemisch aus Heptan und Ethylacetat im Verhältnis 7:3). Man erhält 6,0 g (61 % der Theorie) eines weissen Feststoffs mit einem Schmelzpunkt von 141-149°C.

Elementaranalyse:

| | |
|---|---|
| Berechnet für $C_{49}H_{44}N_4O_6$: | C 75,0; H 5,6; N 7,1 |
| Gefunden : | C 75,1; H 5,8; N 7,0 |

Beispiel 7:

Herstellung von N-Phenyl-3-[(N′,N′-diethylamino)oxy]pyrrolidin-2,5-dion

Die Herstellung erfolgt in Analogie zu Beispiel 1. Es werden 4,46 g (50 mmol) N,N-Diethylhydroxylamin und 8,66 g (50 mmol) N-Phenylmaleimid eingesetzt.

Der Rückstand wird aus Ethanol umkristallisiert und man erhält 6,1 g (46 % der Theorie) eines weissen Feststoffs mit einem Schmelzpunkt von 110-112°C.

Elementaranalyse:

| | |
|---|---|
| Berechnet für $C_{14}H_{18}N_2O_3$: | C 64,1; H 6,9; N 10,7 |
| Gefunden : | C 63,9; H 6,8; N 10,5 |

Beispiel 8:

Herstellung von N-Methyl-3-[(N′,N′-dibenzylamino)oxy]pyrrolidin-2,5-dion

Die Herstellung erfolgt in Analogie zu Beispiel 1. Es werden 5,33 g (25 mmol) N,N-Dibenzylhydroxylamin und 2,78 g (25 mmol) N-Methylmaleimid eingesetzt.

Der Rückstand wird chromatographisch gereinigt (HPLC; Kieselsäuregel; Eluierungsmittel: Gemisch aus Heptan und Ethylacetat im Verhältnis 4:1). Man erhält 7,6 g (94 % der Theorie) einer klaren Flüssigkeit.

Elementaranalyse:

| | |
|---|---|
| Berechnet für $C_{19}H_{20}N_2O_3$: | C 70,4; H 6,2; N 8,6 |
| Gefunden : | C 70,2; H 6,3; N 8,6 |

Beispiel 9:

Herstellung von 3-[(N,N-Dibenzylamino)oxy]pyrrolidin-2,5-dion

Die Herstellung erfolgt in Analogie zu Beispiel 1. Es werden 5,33 g (25 mmol) N,N-Dibenzylhydroxylamin und 2,42 g (25 mmol) Maleimid eingesetzt. Der Rückstand wird chromatographisch gereinigt ((HPLC; Kieselsäuregel; Eluierungsmittel: Gemisch aus Heptan und Ethylacetat im Verhältnis 4:1). Man erhält 5,4 g (74 % der Theorie) eines farblosen Gummis.

Elementaranalyse:

| | |
|---|---|
| Berechnet für $C_{18}H_{18}N_2O_3$: | C 69,7; H 5,8; N 9,0 |
| Gefunden : | C 69,9; H 5,8; N 9,0 |

Beispiel 10:

Herstellung von 1,6-Bis[3-[(N,N-Dibenzylamino)oxy]2,5-dioxopyrrolidin-1-yl]hexan

Die Herstellung erfolgt in Analogie zu Beispiel 1. Es werden 3,08 g (14,5 mmol) N,N-Dibenzylhydroxylamin und 2,0 g (7,2 mmol) 1,6-Dimaleimidohexan eingesetzt.

Beispiel 11:

Verarbeitungsstabilität von Polypropylen

Die Grundformulierung enthält 100 Teile unstabilisiertes Polypropylen (®Profax 6501, Himont) und 0,1 Teil Kalziumstearat. Die unten angegebenen Stabilisatoren werden in Form einer Lösung dem Polypropylen zugemischt (Lösungsmittel: Methylenchlorid). Das Lösungsmittel wird bei vermindertem Druck entfernt. Die stabilisierte Harzformulierung wird bei 100 Umdrehungen pro Minute extrudiert.

Extrusionsbedingungen:

| | |
|---|---|
| Zylinder 1 | 232°C |
| Zylinder 2 | 246°C |
| Zylinder 3 | 260°C |
| Düse 1 | 260°C |
| Düse 2 | 260°C |
| Düse 3 | 260°C |

Nach der ersten, dritten und fünften Extrusion werden die Harz-Kügelchen unter Druck bei 193°C zu 3,2 mm dicken Folien verformt.

Der "Yellowness Index" der Proben wird gemäss ASTM D 1925-63 T bestimmt. Niedrige Werte bedeuten eine geringe Verfärbung. Die Ergebnisse sind in den Tabellen Ia und Ib wiedergegeben.

Der Schmelzindex der Proben wird gemäss ASTM 1238-L bestimmt. Höhere Werte bedeuten eine geringere Molmasse und sind ein Hinweis dafür, dass das Polymer abgebaut ist. Die Ergebnisse sind in Tabelle II aufgeführt.

Tabelle Ia:

| Stabilisator | "Yellowness Index" nach der 1. Extrusion | 3. Extrusion | 5. Extrusion |
|---|---|---|---|
| ohne | 3,6 | 3,9 | 4,6 |
| 0,1 % Antioxidans A | 6,1 | 7,9 | 9,4 |
| 0,1 % Antioxidans A plus 0,05 % Beispiel 1 | 4,2 | 4,9 | 5,3 |
| 0,1 % Antioxidans A plus 0,05 % Beispiel 2 | 3,0 | 4,1 | 6,2 |
| 0,1 % Antioxidans A plus 0,05 % Beispiel 3 | 3,8 | 6,8 | 6,1 |

Tabelle Ib:

| Stabilisator | "Yellowness Index" nach der 1. Extrusion | 3. Extrusion | 5. Extrusion |
|---|---|---|---|
| ohne | 3,8 | 4,0 | 4,4 |
| 0,1 % Antioxidans A | 10,9 | 12,0 | 14,4 |
| 0,1 % Antioxidans A plus 0,05 % Beispiel 6 | 7,7 | 9,1 | 11,0 |
| 0,1 % Antioxidans A plus 0,05 % Beispiel 7 | 7,2 | 8,9 | 9,6 |

Antioxidans A:
Neopentantetrayl-tetrakis[3,5-di-tert-butyl-4-hydroxyhydrocinnamat]

Tabelle II:

| Stabilisator | "Schmelzindex" in g/10 min nach der | |
|---|---|---|
| | 1. Extrusion | 5. Extrusion |
| ohne | 4,4 | 11,5 |
| 0,1 % Antioxidans A | 2,5 | 4,2 |
| 0,1 % Antioxidans A plus 0,05 % Beispiel 1 | 2,1 | 3,2 |
| 0,1 % Antioxidans A plus 0,05 % Beispiel 2 | 2,0 | 3,4 |
| 0,1 % Antioxidans A plus 0,05 % Beispiel 3 | 2,0 | 3,1 |

Antioxidans A:
Neopentantetrayl-tetrakis[3,5-di-tert-butyl-4-hydroxyhydrocinnamat]

Aus den obigen Tabellen ist zu ersehen, dass die erfindungsgemässen Verbindungen wirkungsvolle Stabilisatoren und Farbverbesserer sind.

## Patentansprüche

1. Verbindungen der Formel I,

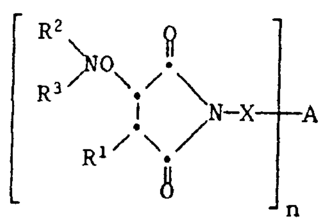

(I)

worin $R^1$ Wasserstoff oder $C_1$-$C_3$-Alkyl bedeutet, $R^2$ und $R^3$ unabhängig voneinander Wasserstoff, $C_1$-$C_{36}$-Alkyl, $C_5$-$C_{12}$-Cycloalkyl, $C_7$-$C_9$-Aralkyloder durch $C_1$-$C_{36}$-Alkyl substituiertes $C_7$-$C_9$-Aralkyl sind, X eine direkte Bindung oder Phenylen ist, n eine ganze Zahl von 1 bis 3 bedeutet und wenn n 1 ist, A Wasserstoff, $C_1$-$C_{30}$-Alkyl, Aryl, $C_5$-$C_8$-Cycloalkyl, $C_7$-$C_9$-Aralkyl, durch $C_1$-$C_{30}$-Alkyl substituiertes $C_7$-$C_9$-Aralkyl oder 2,5-Dioxo-1H-pyrrol-1-yl ist, wenn n 2 bedeutet, A $C_1$-$C_{12}$-Alkylen, Phenylen, $C_6$-$C_{10}$-Cycloalkylen oder Alkylen-Arylen-Alkylen mit

8 bis 10 Kohlenstoffatomen ist und wenn n 3 bedeutet, A Alkantriyl mit 3 bis 6 Kohlenstoffatomen oder

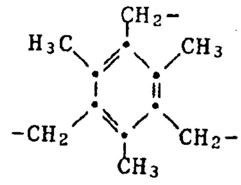

ist.

2. Verbindungen gemäss Anspruch 1, worin $R^1$ Wasserstoff oder Methyl ist und $R^2$ und $R^3$ unabhängig voneinander $C_1$-$C_{18}$-Alkyl, $C_5$-$C_6$-Cycloalkyl, Benzyl, α-Methylbenzyl oder α,α-Dimethylbenzyl bedeuten.

3. Verbindungen gemäss Anspruch 2, worin $R^2$ und $R^3$ Benzyl bedeuten.

4. Verbindungen gemäss Anspruch 1, worin n 1 ist.

5. Verbindungen gemäss Anspruch 4, worin A $C_1$-$C_{18}$-Alkyl, $C_5$-$C_6$-Cycloalkyl, Benzyl, α-Methylbenzyl, α,α-Dimethylbenzyl oder Phenyl bedeutet.

6. Verbindungen gemäss Anspruch 1, worin n 2 ist.

7. Verbindungen gemäss Anspruch 6, worin A $C_1$-$C_6$-Alkylen, Phenylen, Cyclohexylen oder

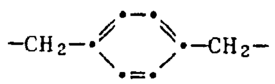

bedeutet.

8. Die Verbindungen
N-Phenyl-3-[(N′,N′-dibenzylamino)oxy]pyrrolidin-2,5-dion,
N-n-Octadecyl-3-[(N′,N′-dibenzylamino)oxy]pyrrolidin-2,5-dion,
1,2-Bis[3-[(N,N-dibenzylamino)oxy]-2,5-dioxopyrrolidin-1-yl]benzol,
1-[3-[(N,N-Dibenzylamino)oxy]-2,5-dioxopyrrolidin-1-yl]-2-[2,5-dioxo-1H-pyrrol-1-yl]benzol,
N-Cyclohexyl-3-[(N′,N′-dibenzylamino)oxy]pyrrolidin-2,5-dion,
1,1-Bis[4-[3-[(N,N-dibenzylamino)oxy]pyrrolidin-2,5-dion-1-yl]phenyl]methan,
N-Phenyl-3-[(N′,N′-diethylamino)oxy]pyrrolidin-2,5-dion,
N-Methyl-3-[(N′,N′-dibenzylamino)oxy]pyrrolidin-2,5-dion,
3-[(N,N-dibenzylamino)oxy]pyrrolidin-2,5-dion und
1,6-Bis[3-[(N,N-dibenzylamino)oxy]-2,5-dioxopyrrolidin-1-yl]hexan gemäss Anspruch 1.

9. Zusammensetzung enthaltend ein gegen oxidativen, thermischen und/oder aktinischen Abbau empfindliches organisches Material und mindestens eine Verbindung gemäss Anspruch 1.

10. Zusammensetzung gemäss Anspruch 9, worin das organische Material ein synthetisches Polymer ist.

11. Zusammensetzung gemäss Anspruch 10, worin das synthetische Polymer ein Polyolefin-Homopolymer oder -Copolymer ist.

12. Zusammensetzung gemäss Anspruch 9, dadurch gekennzeichnet, dass die Zusammensetzung mindestens ein herkömmliches Additiv enthält.

13. Zusammensetzung gemäss Anspruch 12, worin das herkömmliche Additiv ein Metallsalz einer höheren Fettsäure ist.

14. Zusammensetzung gemäss Anspruch 12, worin das herkömmliche Additiv ein phenolisches Antioxidans ist.

15. Zusammensetzung gemäss Anspruch 12, worin die herkömmlichen Additive ein Metallsalz einer höheren Fettsäure und ein phenolisches Antioxidans sind.

16. Zusammensetzung gemäss Anspruch 14, worin das phenolische Antioxidans Neopentantetrayl-tetrakis(3,5-di-tert-butyl-4-hydroxyhydrocinnamat), n-Octadecyl-3,5-di-tert-butyl-4-hydroxyhydrocinnamat, 1,3,5-Trimethyl-2,4,6-tris(3,5-di-tert-butyl-4-hydroxybenzyl)benzol,
1,3,5-Tris(3,5-di-tert-butyl-4-hydroxybenzyl)isocyanurat, 2,6-Ditert-butyl-p-cresol oder 2,2′-Ethyliden-bis(4,6-di-tert-butylphenol) ist.

17. Verwendung von Verbindungen gemäss Anspruch 1 zum Stabilisieren von organischem Material gegen oxidativen, thermischen und/oder aktivischen Abbau.

## Claims

1. A compound of the formula I

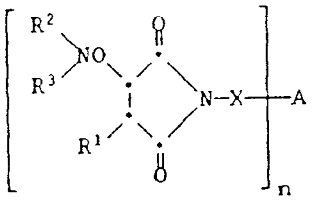

(I)

in which $R^1$ is hydrogen or $C_1$-$C_3$alkyl, $R^2$ and $R^3$ independently are hydrogen, $C_1$-$C_{36}$alkyl, $C_5$-$C_{12}$cycloalkyl, $C_7$-$C_9$aralkyl or $C_1$-$C_{36}$alkyl- substituted $C_7$-$C_9$aralkyl, X is a direct bond or phenylene; n is an integer from 1-3 and when n is 1, A is hydrogen, $C_1$-$C_{30}$alkyl, aryl, $C_5$-$C_8$cycloalkyl, $C_7$-$C_9$aralkyl, $C_1$-$C_{30}$alkyl-substituted $C_7$-$C_9$aralkyl or 2,5-dioxo-1H-pyrrol-1-yl; when n is 2, A is $C_1$-$C_{12}$alkylene, phenylene, $C_6$-$C_{10}$cycloalkylene or alkylenearylenealkylene of 8 to 10 carbon atoms, and when n is 3, A is alkanetriyl of 3 to 6 carbon atoms or

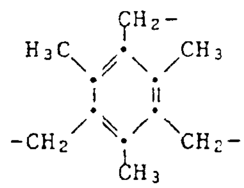

2. A compound of claim 1, in which $R^1$ is hydrogen or methyl and $R^2$ and $R^3$ independently are $C_1$-$C_{18}$alkyl, $C_5$-$C_6$cycloalkyl, benzyl, α-methylbenzyl or α,α-dimethylbenzyl.

3. A compound of claim 2, in which $R^2$ and $R^3$ are benzyl.

4. A compound of claim 1, in which n is 1.

5. A compound of claim 4, in which A is $C_1$-$C_{18}$alkyl, $C_5$-$C_6$cycloalkyl, benzyl, α-methylbenzyl, α,α-dimethylbenzyl or phenyl.

6. A compound of claim 1, in which n is 2.

7. A compound of claim 6, in which A is $C_1$-$C_6$alkylene, phenylene, cyclohexylene or

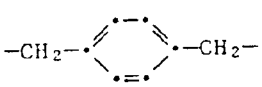

8. A compound of claim 1 selected from among N-phenyl-3-[(N',N'-dibenzylamino)oxy]pyrrolidine-2,5-dione, N-n-octadecyl-3-[CN',N'-dibenzylamino)oxy]pyrrolidine-2,5-dione, 1,2-bis[3-[(N,N-dibenzylamino)oxy]-2,5-dioxopyrrolidin-1-yl]benzene, 1-[3-[(N,N-dibenzylamino)oxy]-2,5-dioxopyrrolidin-1-yl]-2-[2,5-dioxo-1H-pyrrol-1-yl]benzene, N-cyclohexyl-3-[(N',N'-dibenzylamino)oxy]pyrrolidine-2,5-dione, 1,1-bis[4-[3-[(N,N-dibenzylamino)oxy]pyrrolidine-2,5-dion-1-yl]phenyl]methane, N-phenyl-3-[(N',N'-diethylamino)oxy]pyrrolidine-2,5-dione, N-methyl-3-[(N',N'-dibenzylamino)oxy]pyrrolidine-2,5-dione, 3-[(N,N-dibenzylamino)oxy]pyrrolidine-2,5-dione and 1,6-bis[3-[(N,N-dibenzylamino)oxy]-2,5-dioxopyrrolidin-1-yl]hexane.

9. A composition comprising an organic material sensitive to oxidative, thermal and/or actinic degradation and at least one compound of claim 1.

10. A composition of claim 9, in which the organic material is a synthetic polymer.

11. A composition of claim 10, in which the synthetic polymer is a polyolefin homopolymer or copolymer.

12. A composition of claim 9, which contains one or more conventional additives.

13. A composition of claim 12, in which the conventional additive is a metal salt of a higher fatty acid.

14. A composition of claim 12, in which the conventional additive is a phenolic antioxidant.

15. A composition of claim 12 in which the conventional additives are a metal salt of a higher fatty acid and a phenolic antioxidant.

16. A composition of claim 14, in which the phenolic antioxidant is neopentanetetrayl tetrakis-(3,5-di-tert-butyl-4-hydroxyhydrocinnamate), n-octadecyl 3,5-di-tert-butyl-4-hydroxyhydrocinnamate, 1,3,5-trimethyl-2,4,6-tris(3,5-di-tert-butyl-4-hydroxybenzyl)benzene, 1,3,5-tris(3,5-ditert-butyl-4-hydroxybenzyl) isocyanurate, 2,6-di-tert-butyl-p-cresol or 2,2'-ethylidenebis(4,6-di-tert-butylphenol).

17. The use of a compound of claim 1 for stabilizing organic material against oxidative, thermal and/or actinic degradation.

## Revendications

1. Composés de formule I ci-dessous :

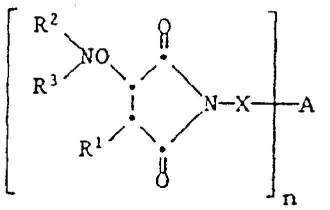

(I)

dans laquelle $R^1$ représente l'hydrogène ou un alkyle en $C_1$-$C_3$, $R^2$ et $R^3$ représentent chacun, indépendamment l'un de l'autre, l'hydrogène, un alkyle en $C_1$-$C_{36}$, un cycloalkyle en $C_5$-$C_{12}$, un aralkyle en $C_7$-$C_9$ ou un tel aralkyle avec un substituant alkylique en $C_1$-$C_{36}$, X représente une liaison directe ou un groupe phénylène, n un entier de 1 à 3 et si n = 1 A est l'hydrogène, un alkyle en $C_1$-$C_{30}$, un aryle, un cycloalkyle en $C_5$-$C_8$, un aralkyle en $C_7$-$C_9$ avec éventuellement un substituant alkylique en $C_1$-$C_{30}$, ou encore un groupe 2,5-dioxo-1H-pyrrol-1-yle, alors que si n = 2 A est un alkylène en $C_1$-$C_{12}$, un phénylène, un cycloalkylène en $C_6$-$C_{10}$ ou un alkylène-arylène-alkylène pouvant avoir de 8 à 10 atomes de carbone, et si n = 3 A est un alcane-triyle avec de 3 à 6 atomes de carbone ou le groupe :

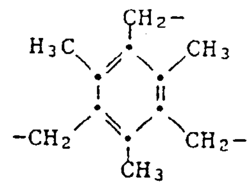

2. Composés selon la revendication 1 dans lesquels $R^1$ est l'hydrogène ou le groupe méthyle, et $R^2$ et $R^3$ sont chacun, indépendamment l'un de l'autre, un alkyle en $C_1$-$C_{18}$, un cycloalkyle en $C_5$-$C_6$, ou le groupe benzyle, $\alpha$-méthylbenzyle ou $\alpha,\alpha$-diméthylbenzyle.

3. Composés selon la revendication 2 dans lesquels $R^2$ et $R^3$ sont le groupe benzyle.

4. Composés selon la revendication 1 dans lesquels n = 1.

5. Composés selon la revendication 4 dans lesquels A est un alkyle en $C_1$-$C_{18}$, un cycloalkyle en $C_5$-$C_6$ ou le groupe benzyle, $\alpha$-méthylbenzyle, $\alpha,\alpha$-diméthylbenzyle ou phényle.

6. Composés selon la revendication 1 dans lesquels n = 2.

7. Composés selon la revendication 6 dans lesquels A est un alkylène en $C_1$-$C_6$ ou bien un groupe phénylène, cyclohexylène ou :

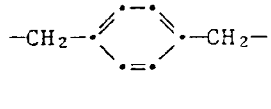

8. Composés suivants conformes à la revendication 1 : N-Phényl-3-[(N,N-dibenzylamino)oxy]-pyrrolidine-2,5-dione, N-n-Octadécyl-3-[(N,N-dibenzylamino)oxy]-pyrrolidine-2,5-dione, 1,2-Bis[3-[(N,N-dibenzylamino)oxy]-2,5-dioxopyrrolidine-1-yl]benzène, 1-[3-[(N,N-Dibenzylamino)oxy]-2,5-dioxopyrrolidine-1-yl]-2-[2,5-dioxo-1H-pyrrol-1-yl]benzène, N-Cyclohexyl-3-[(N,N-dibenzylamino)oxy]pyrrolidine-2,5-dione, 1,1-Bis[4-[3-[(N,N-dibenzylamino)oxy]pyrrolidine-2,5-dione-1-yl]-phényl]méthane, N-Phényl-3-[(N,N-diéthylamino)oxy]pyrrolidine-2,5-dione, N-Méthyl-3-[(N,N-diéthylamino)oxy]pyrrolidine-2,5-dione, 3-[(N,N-dibenzylamino)oxy]pyrrolidine-2,5-dione et 1,6-Bis[3-[(N,N-dibenzylamino)oxy]-2,5-dioxopyrrolidine-1-yl]hexane.

9. Composition qui comprend une matière organique sensible aux dégradations sous l'effet d'une oxydation, de la chaleur et/ou d'un rayonnement actinique, avec un ou plusieurs composés de la revendication 1.

10. Composition selon la revendication 9 dont la matière organique est une matière polymère synthétique.

11. Composition selon la revendication 10 dont la matière polymère synthétique est un homopolymère ou un copolymère d'oléfines.

12. Composition selon la revendication 9, caractérisée en ce qu'elle comprend un ou plusieurs additifs courants.

13. Composition selon la revendication 12 dans laquelle l'additif courant est un sel de métal d'un acide gras supérieur.

14. Composition selon la revendication 12 dans laquelle l'additif courant est un anti-oxydant phénolique.

15. Composition selon la revendication 12 qui comprend comme additifs courants un sel de métal d'un acide gras supérieur et un anti-oxydant phénolique.

16. Composition selon la revendication 14 dans laquelle l'anti-oxydant phénolique est le tétrakis-(3,5-di-tert-butyl-4-hydroxyhydrocinnamante) de néopentane-tétrayle, le 3,5-di-tert-butyl-4-hydroxyhydrocinnamate de n-octadécyle, le 1,3,5-triméthyl-2,4,6-tris-(3,5-di-tertbutyl-4-hydroxybenzyl)-benzène, l'isocyanurate de 1,3,5-tris-(3,5-di-tert-butyl-4-hydroxy-benzyle), le 2,5-di-tertbutyl-p-crésol ou le 2,2'-éthylidène-bis-(4,6-di-tertbutylphénol).

17. Emploi de composés selon la revendication 1 pour stabiliser et protéger des matières organiques contre les dégradations qu'elles peuvent subir par oxydation, sous l'action de la chaleur et/ou sous l'action d'un rayonnement actinique.